# EUROPEAN PATENT SPECIFICATION

(11) **EP 0 952 823 B1**
(45) Date of publication and mention of the grant of the patent: **01.08.2001**
(21) Application number: 97951339.7
(22) Date of filing: 15.12.1997
(51) Int. Cl.: A61K 9/20, A61K 31/40, A61K 47/12

(54) **STABILIZED PHARMACEUTICAL COMPOSITIONS AND PROCESS FOR THE PREPARATION THEREOF**
STABILISIERTE PHARMAZEUTISCHE ZUSAMMENSETZUNGEN UND VERFAHREN ZU IHRER HERSTELLUNG
COMPOSITIONS PHARMACEUTIQUES STABILISEES ET PROCEDE POUR LEUR PREPARATION

(30) Priority: 18.12.1996 HU 9603489
(43) Date of publication of application: 03.11.1999
(73) Proprietor: CHINOIN Gyògyszer és Vegyészeti Termékek Gyára RT., 1045 Budapest IV (HU)
(72) Inventor: TOTH, Antal, H-1118 Budapest (HU); CSERNAK, Lászlú, H-1141 Budapest (HU); KORITSANSZKY, Klára, H-2120 Dunakeszi (HU); SALAMON, Endréné, H-1032 Budapest (HU); VENCZEL, Márta, H-1098 Budapest (HU); VEGELI, Erzsébet, H-1131 Budapest (HU)
(74) Representative: Schwarz, Albin, Dr.
(86) International application number: HU9700084
(87) International publication number: WO9826765

(56) References cited:
- EP-A- 0 468 929
- US-A- 4 793 998

## Description

The present invention relates to novel stabilized pharmaceutical compositions, process for their preparation and the use of maleic acid as a stabilizer. The active ingredient of the above compositions is enalapril maleate which is a potent angiotensin-converting enzyme inhibitor, and it is useful in the treatment of hypertension.

Enalapril, its salts and the process for their preparation are described in the European Patent Application publ. No. EP-012401 A1.

As it is known, many compounds that inhibit ACE (Angiotensin-Converting Enzyme) have poor stability either in form of free acids or salts if they are in a pharmaceutical dosage form. These compounds easily decompose first of all by hydrolysis and intramolecular cyclization, but the amount of other decomposition products not identified in many cases may be also significant. This is particularly true in case of enalapril and its maleate salt.

Main decomposition products of enalapril are shown in Fig. demonstrating that the decomposition is due to hydrolytic and cyclization processes.

The diketopiperazine (DPK) is the internal cyclization product and the diacid (enalaprilat ET) is the product of ester hydrolysis.

A lot of solutions have been elaborated to stabilize angiotensin-converting enzyme inhibitors, among them enalapril salts in pharmaceutical compositions.

According to the European Patent Application publ. no. EP-0 280 999 A2, magnesium carbonate shows a stabilizing effect in pharmaceutical products containing saccharides, e.g. lactose and quinapril.

According to the European Patent Application publ. no. EP-0 545 194 A1, enalapril is transformed into its sodium salt. The enalapril sodium salt in pharmaceutical preparations is said to be more stable than the enalapril maleate salt.

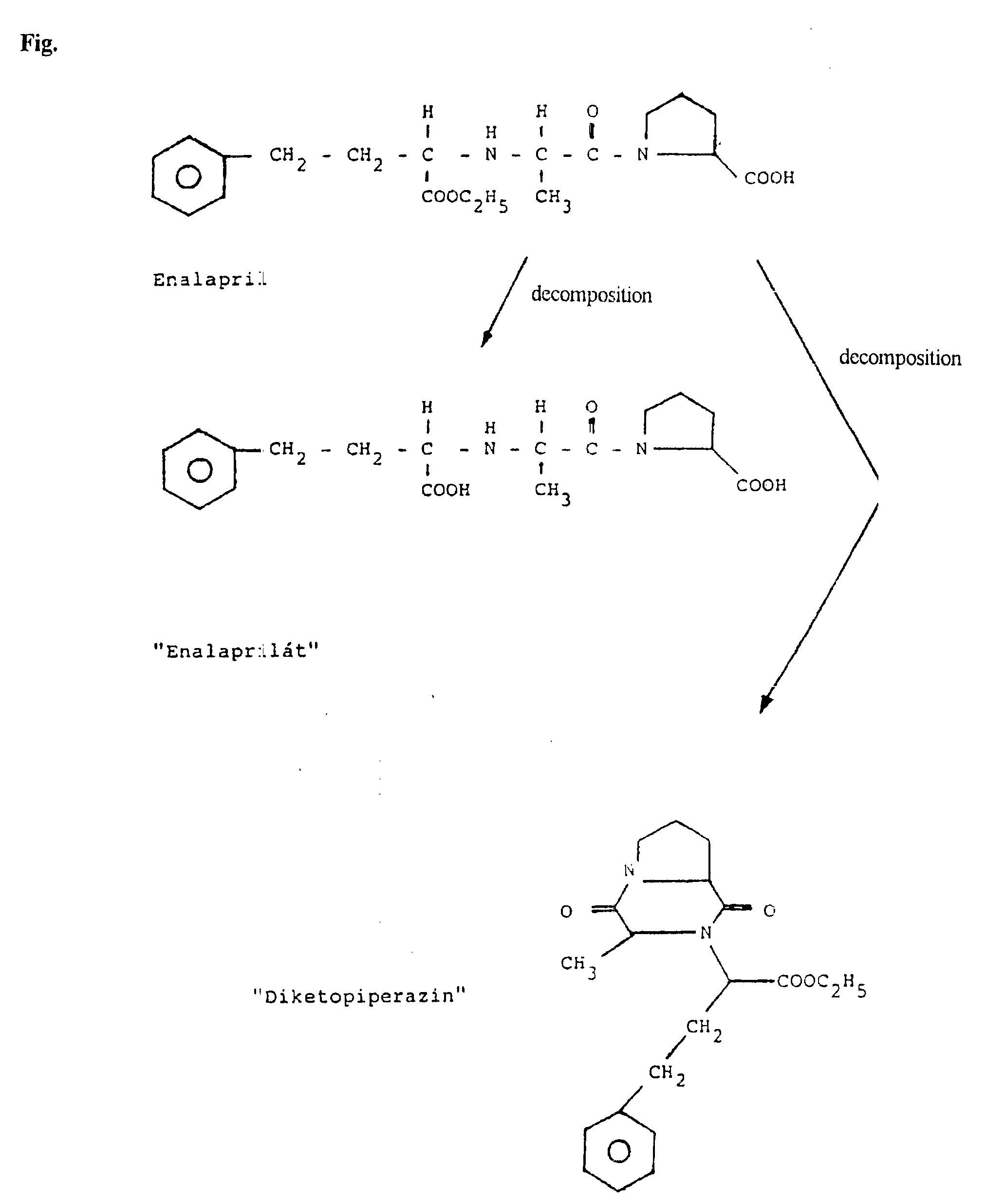

United States Patent no. 5.562.921 describes that the enalapril maleate salt extensively decomposes in the presence of commonly used vehicles, filling substances, lubricants or disintegrating agents in many pharmaceutical products for example in the pharmaceutical dosage forms containing microcrystalline cellulose, calcium phosphate or magnesium stearate.
It is described in EP-A-099239 and EP-B-0264887 that ascorbic acid may be used as an antioxidant or colour stabilizing agent in case of ACE-inhibitors.

The aim of our invention is to prepare pharmaceutical formulations of high stability which contain enalapril maleate with commonly used filling substances (e.g. lactose, mannitol, sorbitol) lubricant (e.g. magnesium stearate) and disintegrating agents (e.g. starch) and in which the amount of decomposition products is low even in case of long-term storage, thus ensuring a longer expiration time and in the same time a high quality.

It has been found that if enalapril maleate is transformed into pharmaceutical formulations by applying commonly used filling substance (e.g. filling substance of saccharide type) and maleic acid stabilizer, an extremely stable enalapril formulation is obtained. This is true even if magnesium stearate or other compounds are used as lubricants, affecting the stability of enalapril maleate .

At realizing our invention, we have successfully applied for example mono- or disaccharides, water-free lactose, lactose monohydrate or DC (direct compression) lactose as filling substances, starches or partly hydrolysed starches, or crospovidone (polyvinylpolypyrrolidone) as disintegrating agents, stearate salts or esters such as magnesium stearate, hydrogenated vegetable oil or talc as lubricants, and maleic acid as stabilizer, in addition to the currently used colouring and binding agents, e.g. ferric oxide and povidone (polyvinylpyrrolidone). Further auxiliary substances applicable for these purposes are enumerated in the Hungarian Pharmacopoeia or in the European Pharmacopoeia.

Prefered variant forms are tablets or capsules, particularly tablets having 75-300 mg of tablet mass.

One of the preferred variant forms of our invention is the tablet or the granules for filling capsule consisting of enalapril maleate, maleic acid, lactose, starch, partly hydrolysed starch and magnesium stearate, and optionally colouring and binding agents.

Our invention also relates to the process for the preparation of the above pharmaceutical formulations. During this process, granules to be compressed in tablets or to be filled into capsules are prepared by wet granulation using aqueous solution of maleic acid.

During one of the favourable implementations of the above process, dry enalapril maleate, lactose, starch and partly hydrolysed starch are mixed, and then their mixture is granulated using aqueous solution of maleic acid used as granulation liquid. Of course, ingredients may be mixed in other sequences. The granules obtained are dried and classified, and then compressed into tablets with magnesium stearate added.

Tablets according to our invention can be prepared by direct tabletting, i.e. mixing enalapril maleate with all other auxiliary substances and with maleic acid used as stabilizing agent, and by compressing the mixture in tablets.

Pharmaceutical products (dosage forms) prepared according to our invention may preferably contain enalapril maleate in 0.1-25 weight %, lactose in 30-95 weight %, starch and partly hydrolysed starch in 6-80 weight %, maleic acid in 0.1-10 weight %, lubricant in 0.1-5 weight % and colouring and binding agents in 0.01-5 weight %.

A preferred composition is disclosed in claim 2.

Preferred dosage forms are tablets and granules which contain enalapril maleate in 1.5-15 weight %, lactose in 65-90 weight %, starch and/or other disintegrating agents in 5-15 weight %, binding and colouring agents in 3-7 weight %, pregelatinized starch in 1-4 weight %, maleic acid in 1-5 weight % and lubricants in 0.1-1.5 weight %. Most preferred unit dosage forms are tablets with 75-300 mg tablet mass having above preferred compositions.

The present invention also concerns a composition as disclosed in claim 9.

Further details of our invention are shown by the examples below, without limiting our claims to the examples.

### Examples

### Example 1

100 g of enalapril maleate, 3930 g of lactose monohydrate, 380 g of corn starch, 120 g of pregelatinized starch were homogenized. 24 g of maleic acid was dissolved in 1000 ml of purified water. The homogenized powder mixture was granulated with slowly (10-15 min) added aqueous solution of maleic acid. The wet granules were dried (at 40-50°). The dried granules were homogenized with 20 g of magnesium stearate (for 10-20 min).
The homogenized granules were tabletted and tablets having 115 mg total mass and containing 2.5 mg of enalapril maleate were obtained.

### Example 2

150 g of enalapril maleate, 5934 g of lactose monohydrate, 570 g of corn starch, 180 g of pregelatinized starch were homogenized. 36 g of maleic acid was dissolved in 1500 ml of purified water. The homogenized powder mixture was granulated with slowly (10-15 min) added aqueous solution of maleic acid. The wet granules were dried (at 40-50°). The dried granules were homogenized with 30 g of magnesium stearate (for 10-20 min).
The homogenized granules were tabletted.

### Example 3

200 g of enalapril maleate, 3300 g of lactose monohydrate, 300 g of corn starch, 100 g of pregelatinized starch were homogenized. 48 g of maleic acid was dissolved in 950 ml purified water. The homogenized powder mixture was granulated by slowly (10-15 min) added aqueous solution of maleic acid. The wet granules were dried (at 40-50°). The dried granules were homogenized with 36 g of magnesium stearate (for 10-20 min).
The homogenized granules were tabletted.

### Example 4

250 g of enalapril maleate, 1890 g of lactose monohydrate, 188 g of corn starch, 68 g of pregelatinized starch were homogenized. 60 g of maleic acid was dissolved in 600 ml of purified water. The homogenized powder mixture was granulated with slowly (10-15 min) added aqueous solution of maleic acid. The wet granules were dried (at 40-50°). The dried granules were homogenized with 45 g of magnesium stearate (for 10-20 min).
The homogenized granules were tabletted and tablets having 200 mg total mass and containing 20 mg of enalapril maleate were obtained.

### Example 5

200 g of enalapril maleate, 3300 g of lactose monohydrate, 300 g of corn starch, 100 g of pregelatinized starch, 48 g of maleic acid were homogenized. The homogeneous powder mixture was granulated with slowly (10-15 min) added purified water (950 ml). The wet granules were dried (at 40-50°). The dried granules were homogenized with 36 g of magnesium stearate (for 10-20 min).
The homogenized mixture was tabletted.

### Example 6

3300 g of lactose monohydrate, 300 g of corn starch, 100 g of pregelatinized starch were homogenized. 48 g of maleic acid was dissolved in 1100 ml of purified water. While stirring, 200 g of enalapril maleate was added. The homogeneous powder mixture was added to the suspension. The wet granules were dried (at 40-50°). The dried granules were homogenized with 36 g of magnesium stearate.
The homogenized granules were tabletted.

### Example 7

250 g of enalapril maleate; 4200 g of lactose monohydrate, 370 g of corn starch, 120 g of pregelatinized starch were homogenized. 45 g of maleic acid was dissolved in 1300 ml of purified water. While stirring, 120 g of polyvinylpyrrolidone was added to the pure solution. The homogenized powder mixture was granulated with slowly (10-15 min) added aqueous solution of maleic acid and polyvinylpyrrolidone. The wet granules were dried (at 40-50°). The dried granules were homogenized with 36 g of magnesium stearate (for 10-20 min).
The homogenized granules were tabletted.

### Example 8

250 g of enalapril maleate, 60 g of maleic acid, 45 g of magnesium stearate, 120 g of polyvinylpyrrolidone, 120 g of pregelatinized starch were homogenized. 370 g of corn starch, 4200 g of lactose monohydrate were added to the homogeneous mixture and the mixture was homogenized again (for 15-20 min).
The homogeneous mixture was tabletted.

### Example 9

200 g of enalapril maleate, 1600 g of lactose monohydrate, 1600 g of corn starch, 250 g of pregelatinized starch, 100 g of polyvinylpyrrolidone, 150 g of talc were homogenized. 50 g of maleic acid was dissolved in 1000 ml of purified water. The homogenized powder mixture was granulated with slowly (10-15 min) added aqueous solution of maleic acid. The wet granules were dried (at 40-50°). The dried granules were homogenized with 36 g of magnesium stearate.
The homogenized granules were tabletted.

### Example 10

200 g of enalapril maleate, 250 g of pregelatinized starch were homogenized, 100 g of polyvinylpyrrolidone, 150 g of talc, 50 g of maleic acid and 40 g of magnesium stearate were homogenized. To the homogeneous mixture 1600 g of lactose and 1600 g of corn starch were
added. The mixture was homogenized (for 15-20 min).
The homogenized mixture was tabletted.

### Example 11

100 g of enalapril maleate, 1700 g of lactose monohydrate, 40 g of crospovidone, 110 g of maize starch and 2 g of ferrous oxide red were homogenized. 48 g of maleic acid was dissolved in 1200 ml of purified water. The homogenized powder mixture was granulated with slowly (10-15 min) added aqueous solution of maleic acid. The wet granules were dried at 40-50°C. The dried granules were homogenized with 10 g of magnesium stearate for 20 min. The homogenized granules were tabletted and tablets having 200 mg total mass and containing 10 mg of enalapril maleate were obtained.

## Claims

1. Stable pharmaceutical composition, **characterized** in that, it contains enalapril maleate as active substance, maleic acid as stabilizing agent and one or more auxiliary substances.

2. Composition according to claim 1, **characterized** in that, it contains enalapril maleate in 1.5-15 weight %, filling substances in 65-90 weight %, disintegrating agents in 6-20 weight %, maleic acid in 1-5 weight %, binding and colouring agents in 3-7 weight % and lubricants in 0.1-1.5 weight %.

3. Composition according to claim 1, **characterized** in that, it contains mono- or disaccharides as filling substance, starches and/or crospovidone as disintegrating agent, stearate salts or esters, or hydrogenated vegetable oils as lubricants.

4. Composition according to claim 1, **characterized** in that, it contains enalapril maleate as active substance, maleic acid as stabilizing agent, lactose as filling substance, starch and crospovidone as disintegrating agent, magnesium stearate, hydrogenated vegetable oil or talc as lubricant, povidone as binding agent and optionally ferric oxide as colouring agent.

5. Composition according to claim 1, **characterized** in that, it contains enalapril maleate as active substance, maleic acid as stabilizing agent, lactose monohydrate as filling substance, starch as disintegrating agent, magnesium stearate as lubricant and optionally ferric oxide as colouring agent.

6. Composition according to claim 1, **characterized** in that, the dosage form is a tablet.

7. Composition according to claim 6, **characterized** in that, the dosage from is a tablet having 75-300 mg of tablet mass.

8. Composition according to claim 1, characterized in that, the dosage form is a capsule filled with granules.

9. Composition according to claim 1, **characterized** in that, it is in a commercial package in the form of orally applicable dosage form together with instructions for its administering.

10. Process for the preparation of composition according to claim 1, **characterized** in that, wet granulation is used.

11. Process according to claim 10, **characterized in** that, wet granulation is carried out with aqueous solution of maleic acid used as stabilizing agent.

12. Process according to claim 10 **characterized in** that, the enalapril maleate, lactose, disintegrating and colouring agents are mixed, dried, aqueous solution of stabilizing maleic acid is added, the mixture is wet granulated, the obtained granules are dried, classified, mixed with lubricant and compressed into tablets.

13. Use of maleic acid to stabilize enalapril maleate in a pharmaceutical composition as defined in anyone of claims 1 to 8.

14. Use of maleic acid as stabilizing agent in the manufacture of a pharmaceutical composition containing enalapril maleate as defined in anyone of claims 1 to 8.

## Patentansprüche

1. Stabile pharmazeutische Zusammensetzung, **dadurch gekennzeichnet**, dass sie Enalaprilmaleat als Wirkstoff, Maleinsäure als Stabilisierungsmittel und einen oder mehrere Hilfsstoffe enthält.

2. Zusammensetzung nach Anspruch 1, **dadurch gekennzeichnet**, dass sie Enalaprilmaleat in einer Menge von 1,5 - 15 Gew.-%, Füllstoffe in einer Menge von 65 - 90 Gew.-%, Aufschlussmittel in einer Menge von 6 - 20 Gew.-%, Maleinsäure in einer Menge von 1 - 5 Gew.-%, Binde- und Färbemittel in einer Menge von 3 - 7 Gew.-% und Schmierstoffe in einer Menge von 0,1 - 1,5 Gew.-% enthält.

3. Zusammensetzung nach Anspruch 1, **dadurch gekennzeichnet**, dass sie Mono- oder Disaccharide als Füllstoff, Stärken und/oder Crospovidon als Aufschlussmittel, Stearatsalze oder -ester oder gehärtete pflanzliche Öle als Schmierstoffe enthält.

4. Zusammensetzung nach Anspruch 1, **dadurch gekennzeichnet**, dass sie Enalaprilmaleat als Wirkstoff, Maleinsäure als Stabilisierungsmittel, Lactose als Füllstoff, Stärke und Crospovidon als Aufschlussmittel, Magnesiumstearat, gehärtetes pflanzliches Öl oder Talk als Schmierstoff, Povidon als Bindemittel und gegebenenfalls Eisenoxid als Färbemittel enthält.

5. Zusammensetzung nach Anspruch 1, **dadurch gekennzeichnet**, dass sie Enalaprilmaleat als Wirkstoff, Maleinsäure als Stabilisierungsmittel, Lactosemonohydrat als Füllstoff, Stärke als Aufschlussmittel, Magnesiumstearat als Schmierstoff und gegebenenfalls Eisenoxid als Färbemittel enthält.

6. Zusammensetzung nach Anspruch 1, **dadurch gekennzeichnet**, dass die Dosisform eine Tablette ist.

7. Zusammensetzung nach Anspruch 6, **dadurch gekennzeichnet,** dass die Dosisform eine Tablette mit 75 - 300 mg Tablettenmasse ist.

8. Zusammensetzung nach Anspruch 1, **dadurch gekennzeichnet**, dass die Dosisform eine mit Granulat gefüllte Kapsel ist.

9. Zusammensetzung nach Anspruch 1, **dadurch gekennzeichnet**, dass sie in einer handelsüblichen Packung in der Form einer oral anwendbaren Dosisform zusammen mit Anweisungen für ihre Verabreichung vorliegt.

10. Verfahren zur Herstellung der Zusammensetzung nach Anspruch 1, **dadurch gekennzeichnet**, dass eine Feuchtgranulierung eingesetzt wird.

11. Verfahren nach Anspruch 10, dadurch gekennzeichnet, dass die Feuchtgranulierung unter Verwendung einer wässerigen Lösung von Maleinsäure als Stabilisierungsmittel durchgeführt wird.

12. Verfahren nach Anspruch 10, **dadurch gekennzeichnet**, dass das Enalaprilmaleat, die Lactose, die Aufschluss- und Färbemittel gemischt und getrocknet werden, die wässerige Lösung von stabilisierender Maleinsäure zugesetzt wird, die Mischung feuchtgranuliert wird und das erhaltene Granulat getrocknet, klassiert, mit einem Schmierstoff gemischt und zu Tabletten gepresst wird.

13. Verwendung von Maleinsäure zum Stabilisieren von Enalaprilmaleat in einer pharmazeutischen Zusammensetzung wie in einem der Ansprüche 1 bis 8 definiert.

14. Verwendung von Maleinsäure als Stabilisierungsmittel bei der Herstellung einer Enalaprilmaleat enthaltenden pharmazeutischen Zusammensetzung wie in einem der Ansprüche 1 bis 8 definiert.

## Revendications

1. Composition pharmaceutique stable, caractérisée en ce qu'elle contient du maléate d'énalapril en tant que principe actif, de l'acide maléique en tant qu'agent stabilisant et une ou plusieurs substances auxiliaires.

2. Composition selon la revendication 1, caractérisée en ce qu'elle contient 1,5 à 15 % en masse de maléate d'énalapril, 65 à 90 % en masse de substances de charge, 6 à 20 % en masse d'agents de délitement, 1 à 5 % en masse d'acide maléique, 3 à 7 % en masse d'agents liants et colorants et 0,1 à 1,5 % en masse de lubrifiants.

3. Composition selon la revendication 1, caractérisée en ce qu'elle contient des mono- ou disaccharides en tant que substances de charge, des amidons et/ou de la crospovidone en tant qu'agents de délitement, des sels ou des esters de stéarate ou des huiles végétales hydrogénées en tant que lubrifiants.

4. Composition selon la revendication 1, caractérisée en ce qu'elle contient du maléate d'énalapril en tant que principe actif, de l'acide maléique en tant qu'agent stabilisant, du lactose en tant que substance de charge, de l'amidon et de la crospovidone en tant qu'agent de délitement, du stéarate de magnésium, une huile végétale hydrogénée ou du talc en tant que lubrifiant, de la povidone en tant qu'agent liant et éventuellement de l'oxyde ferrique en tant qu' agent colorant.

5. Composition selon la revendication 1, caractérisée en ce qu'elle contient du maléate d'énalapril en tant que principe actif, de l'acide maléique en tant qu'agent stabilisant, du lactose monohydraté en tant que substance de charge, de l'amidon en tant qu'agent de délitement, du stéarate de magnésium en tant que lubrifiant et éventuellement de l'oxyde ferrique en tant qu'agent colorant.

6. Composition selon la revendication 1, caractérisée en ce que la forme pharmaceutique est un comprimé.

7. Composition selon la revendication 6, caractérisée en ce que la forme pharmaceutique est un comprimé ayant une masse de 75 à 300 mg.

8. Composition selon la revendication 1, caractérisée en ce que la forme pharmaceutique est une gélule remplie de granulés.

9. Composition selon la revendication 1, caractérisée en ce qu'elle est dans un conditionnement commercial sous la forme d'une forme pharmaceutique destinée à une administration orale, accompagnée des instructions pour son administration.

10. Procédé pour la préparation de la composition selon la revendication 1, caractérisé en ce que l'on utilise une granulation par voie humide.

11. Procédé selon la revendication 10, caractérisé en ce que la granulation par voie humide est réalisée avec une solution aqueuse d'acide maléique utilisé en tant qu'agent stabilisant.

12. Procédé selon la revendication 10, caractérisé en ce que l'on mélange le maléate d'énalapril, le lactose, les agents colorant et de délitement, on sèche, on ajoute une solution aqueuse d'acide maléique stabilisant, on granule le mélange par voie humide, on sèche les granulés obtenus, on les tamise, on les mélange au lubrifiant et on les comprime pour obtenir des comprimés.

13. Utilisation de l'acide maléique pour stabiliser le maléate d'énalapril dans une composition pharmaceutique telle que définie dans une quelconque des revendications 1 à 8.

14. Utilisation de l'acide maléique en tant qu'agent stabilisant dans la fabrication d'une composition pharmaceutique contenant du maléate d'énalapril telle que définie dans une quelconque des revendications 1 à 8.
